# EUROPEAN PATENT SPECIFICATION

(11) **EP 0 863 118 B1**
(45) Date of publication and mention of the grant of the patent: **21.11.2001**
(21) Application number: 98250027.4
(22) Date of filing: 28.01.1998
(51) Int. Cl.: C07C 17/12, C07C 25/22

(54) **Preparation of Brominated Indanes**
Verfahren zur Herstellung von bromierten Indanen
Procédé pour la préparation d'indanes bromés

(30) Priority: 07.03.1997 US 811770
(43) Date of publication of application: 09.09.1998
(73) Proprietor: ALBEMARLE CORPORATION, Baton Rouge, LA 70801-1765 (US)
(72) Inventor: Hussain, Saadat, Baton Rouge, LA 70817 (US)
(74) Representative: UEXKÜLL & STOLBERG

(56) References cited:
- EP-A- 0 571 036

## Description

This invention relates generally to the bromination of aromatic ring compounds to prepare products which are useful as flame retardants in plastics. More particularly, the invention relates to an improved process for the manufacture of brominated indanes, such as phenyl indanes, of high purity.

Polybrominated phenyl indanes and fire retardant polymeric compositions containing such compounds are described in European Patent Application No. 571,036. The polybrominated phenyl indanes are prepared by mixing a phenyl indane, such as 1,3,3-trimethyl-1-phenyl indane, and bromine in a solvent using a metal or metal halide Lewis acid catalyst such as iron or aluminum chloride. During this process, undesirable impurities are produced due to cleavage of the 5-membered ring at the benzylic carbon. Also, the product contains 10 percent or more of partially brominated materials (Br₅₋₇ isomers) even after recrystallization. This reduces yields and can introduce color bodies into the product.

An improved solvent bromination process has now been found which produces polybrominated indanes in high yields while minimizing the production of undesirable impurities and/or partially brominated materials such that high purity (>95%) products are obtained without the need for recrystallization.

In accordance with this invention, there is provided a process for ar-brominating 1,3,3-trimethyl-1-phenyl indane to produce octabrom-1,3,3-trimethyl-1-phenyl indane, said process comprising:
(a) adding said 1,3,3-trimethyl-1-phenyl indane to an organic solvent solution of bromine in the presence of an ar-bromination catalyst at a rate such that the addition is complete within about 1.5 hours, with the proportions of solvent to 1,3,3-trimethyl-1-phenyl indane being selected such that the weight ratio of the former to the latter is less than 20 grams of solvent per gram of 1,3,3-trimethyl-1-phenyl indane, and then
(b) heating the reaction mixture to a temperature of at least about 90°C so as to produce said octabromo-1,3,3-trimethyl-1-phenyl indane.

As used herein, ar-bromination, ar-bromine, and poly-ar-brominated, all relate to bromine substitution on an aromatic carbon. This is to distinguish from bromine addition to an alkyl carbon.

Also provided are an efficient process for recycling the reaction mixture to the process for re-use after the product has been removed and a brominated phenyl indane product which has a purity of octabromophenyl indane of at least 92% by weight, and usually 95% or more by weight, without recrystallization.

The 1,3,3-trimethyl-1-phenyl indane can be prepared by the dimerization of alpha-methylstyrene as is known in the art.

It is preferred that the bromine used in the process of this invention be essentially anhydrous, i.e., contain less than 100 ppm water, and contain no more than 10 ppm by weight of organic impurities, e.g., oil, grease, carbonyl containing hydrocarbons, and iron. With such a bromine purity, there is little, if any, impact on the color attributes of the polybrominated indane product Available, commercial grade bromine may have such purity. If, however, such is not available, the organic impurities and water content of the bromine can be conveniently reduced by mixing together a 3 to 1 volume ratio of bromine and concentrated (94 - 98 percent) sulfuric acid. A two-phase mix is formed which is stirred for 10 - 16 hours. After stirring and settling, the sulfuric acid phase, along with the impurities and water, is separated from the bromine phase. To further enhance the purity of the bromine, the recovered bromine phase can be subjected to distillation.

The organic solvent medium used for the bromination process should be substantially inert to the reactants and anhydrous. Preferred solvents are halogenated, saturated aliphatic hydrocarbons such as lower alkyl halides, for example, carbon tetrachloride. chloroform, tetrachloroethane, methylene chloride, trichloroethane, dibromoethane, and dibromomethane. Examples of other solvents that can be used include, ethylene dichloride (EDC), bromochloromethane and tetrachloroethylene. Mixtures of solvents can be used.

In order to obtain high yields of fully brominated indanes in accordance with the process of this invention, the solvent to indane reactant ratio should be less than 20 grams of solvent per gram of indane compound and, preferably, from 10 to 18 grams of solvent per gram of indane.

The amount of bromine used is, preferably, in slight excess (e.g., 10 to 25%) of the stoichiometric amount needed to ar-brominate the indane compound to the desired extent. Preferably, the indane compound will be completely ar-brominated. For example, in the case of a phenyl indane, an octabromophenyl indane would be produced. Such high bromine content materials are preferred for use as flame retardant additives in plastics. Although the phenyl indanes have up to nine aromatic ring carbon atoms available for substitution, one of the carbon atoms on the phenyl group is sheltered by steric effects due to the folded configuration of the phenyl indane molecule and so usually remains unsubstituted. In order to provide the ar-perbromination needed to obtain an octabrominated product, a strong Lewis acid bromination catalyst system is used.

Aluminum or iron containing bromination catalysts are preferred, e.g., AlCl₃, FeBr₃, and/or AlBr₃, although use may be made of aluminum powder, iron powder, FeCl₃, FeBr₃, and ZrCl₄, either alone or in combination with the aluminum trihalides. Using an iron containing catalyst usually provides a colored product. Other strong Lewis acid bromination catalysts may be suitable, provided that they have sufficient catalytic activity to provide for the ar-bromination needed. Catalytic quantities are used. Typically, the catalyst will be present in an amount within the range of from 0.1 to 20 weight percent, based on the weight of the indane reactant. The amount used will generally depend on the catalytic activity of the chosen catalyst, the reaction temperature, and the amount of bromine used. A preferred amount is within the range of from 2 to 15 weight percent based on the weight of indane. When AlCl₃ is the catalyst, for example, from 3.0 to 5.0 weight percent will be most preferred.

The bromination catalyst, bromine and solvent can be charged to the reactor in any order or together. In the initial cycle of a recycle process, the indane compound can be fed to the reactor either as a solution in the inert organic reaction solvent or as a solid or molten feed. In the further cycles of a recycle process, the indane compound is preferably fed neat, either as a solid or molten, in order to avoid the dilution of the reaction with additional solvent. In such case, it is preferred that the indane feed be molten and be introduced under the surface of the reaction mixture such as by using a dip tube. This provides more bromination due to faster rates and better mixing. However, the molten indane can also be fed into the reaction mixture from above.

In order to achieve a good yield of high purity product, it is important that the addition rate be such that the indane addition time is less than 1.5 hours. Prior art slower addition rates are believed to result in ar-brominated products which contain 10% by weight more of underbrominated materials. The rate of addition should maintain the reaction temperature at 30° C or above throughout the addition. Following the addition of the indane compound, the reaction mixture is heated to a temperature of at least 90° C until the reaction is completed as indicated by the ceasing of HBr evolution. Temperatures of from 90° C to 100° C or above can be used.

The brominated indane product will separate as a solid from the reaction mixture and can be recovered by conventional techniques such as filtration. The remaining reaction mixture (i.e., the filtrate) can then be directly recycled, in accordance with a further aspect of the invention, to the reactor without the neutralization of and removal of the excess bromine or the catalyst. Additional bromine is added to provide the desired stoichiometric amount for the next cycle of the reaction with the fresh indane reactant This process permits the re-use of the catalyst and excess bromine and avoids the need to dispose of the neutralized waste after each cycle as in the prior art process. As previously mentioned, the indane compound is preferably melted and added to the reaction such that required solvent to indane compound weight ratio of less than 20/1 is achieved. Additional catalyst is also added as required. Recycle of the filtrate from the previous batch cannot be continued endlessly, as the quality of the product (especially the color) will start to suffer due to the build-up of impurities. After 4 - 5 recycles, the filtrate needs to be treated for removal and disposal of undesirable impurities. At this point, a fresh batch utilizing fresh solvent and bromine, as well as the catalyst is utilized.

The invention is further illustrated by, but not intended to be limited to, the following examples.

### EXAMPLE 1

The reactor is a 1 liter, 4-necked flask equipped with a mechanical stirrer, a thermometer with a therm-o-watch, an addition funnel, a reflux condenser with an assembly to exit HBr/Br₂ fumes to a caustic scrubber, and a heating mantle. The reactor is charged with bromine (66.2 mL, 205.2 g, 1.282 moles), dibromomethane solvent (200 mL) and iron powder (0.9 g) in that order. A solution of 1,3,3-trimethyl-1-phenyl indane (TMPI) (30.0 g, 0.127 mole) in 20 mL of dibromomethane is prepared and charged to the addition funnel. The mole ratio of bromine to TMPI is 10.1/1. The TMPI solution is added to the stirred bromine/solvent/iron slurry, dropwise. over a period of 35 minutes. The temperature during addition is from 35° C to 38° C. After the addition is complete, the reaction mixture is heated to 75° C for 1.5 hours. The scrubber gain is 78.5 g of HBr (vs 82.3 by theory). At this point, the reaction mixture is cooled, another 0.5 g of iron powder is added and then the reaction mixture is heated back to 75° C and kept at that temperature for 1 hour. The reaction mixture is then cooled, 250 mL of water is added followed by an aqueous solution of sodium metabisulfite in 100 mL of water and then another 10.8 g of solid sodium metabisulfite to react with the remaining bromine. The reaction slurry is transferred to a 1-liter separatory funnel where the layers separate and the organic layer is removed and filtered through a coarse porosity, sintered glass funnel using gravity until all of the solvent is filtered. Vacuum is then applied to remove the remaining liquids. The filter cake is washed consecutively with water (2 x 100 mL), aqueous sodium carbonate (10 g/100 mL water), water (2 x 100 mL) and then acetone (2 x 100 mL). The acetone wash is green. The solids are dried overnight. The yield of light tan-off white crystalline solid product is 70.3 g (0.081 mole, 63.8%, m.pt 248 - 252° C). The purity of the octabromo-1,3,3-trimethyl-1-phenylindane by GC analysis is 96.4 area % with the major impurity being the Br₇ isomer.

### EXAMPLE 2

A 3 liter, 3-necked flask equipped with the same accessories as in Example 1 is charged with bromine (398 mL, 10.1 moles per mole of TMPI used), followed by iron powder (5.4 g) and dibromomethane solvent (1170 mL). The addition funnel is charged with a solution of TMPI (180 g, 0.763 mole) in 150 mL of dibromomethane solvent. The TMPI/dibromomethane solution is added to the stirred Br₂/CH₂Br₂/Fe slurry, dropwise, at a temperature of 30 - 40° C over a period of 55 minutes. The reaction mixture is stirred for another 5 minutes at ambient and is then heated. Reflux begins at 50° C with very vigorous HBr evolution occurring. The temperature rises to 94° C after 70 minutes and the heat is then cut and the reaction mixture is allowed to cool. Water is added (50 mL) followed by an aqueous solution of sodium metabisulfite (75 g in 250 mL water) with stirring. Most Br₂ is destroyed but some orange color is left. Another 25.3 g of solid sodium metabisulfite is added with stirring. The reaction slurry temperature reaches 65° C during the water and sodium metabisulfite treatments due to the exotherm. The slurry is allowed to cool to ambient with stirring. The top aqueous layer is highly colored. Part of the aqueous layer is decanted and the rest of the slurry is filtered without separating the organic layer from the remaining aqueous phase. The filter cake (dark) is washed with water (2 x 225 mL), dibromomethane (2 x 100 mL) and then acetone (5 x 200 mL). The acetone washes remove all of the color and the filter cake is off-white. The cake is allowed to dry in air overnight. The yield of dry product is 405.7 g. More solids precipitate from the dibromomethane wash and are recovered by filtration and washed with acetone (5 x 100 mL) followed by drying at 100° C for 20 minutes. The additional yield of product is 54.1 g of off-white solid. The total yield of octabromo-1.3,3-trimethyl-1-phenylindane is 459.8 g (69.4%) whose purity by G.C. analysis is 99.3 area %.

### EXAMPLE 3

A 3-L 3-necked round bottom flask equipped with a mechanical stirrer, a thermometer, a glycol-cooled reflux condenser, an addition funnel and a caustic scrubber is charged with bromine (398 mL, 10.1 moles per mole of TMPI used), followed by iron powder (5.4 g) and dibromomethane solvent (1170 mL). The addition funnel is charged with a solution of TMPI (180 g, 0.763 mole) in 150 mL of dibromomethane solvent. With stirring, the TMPI/dibromomethane solution is added to the stirred Br₂/CH₂Br₂/Fe slurry, dropwise, at a temperature of 30° - 40° C, over a period of sixty minutes. The reaction temperature is now allowed to rise at a rate to maintain the reflux. The reflux begins at 50° C initially, and rises to 94° C over a period of 1.5 hours, as more and more bromine is consumed in the reaction. The reaction mixture is maintained at 94° C for 15 - 30 minutes, then allowed to cool to 50° C and filtered directly. The filtrate is stored in an air-tight (under nitrogen) container for recycling into the next batch. The solid is washed with water (2 x 200 mL) and then with acetone (5 x 200 mL). The filtrate from these washes is disposed of. The solid is dried at 125° C for two hours in an oven to give a white octabrominated product in an expected yield of 70%.

In the next cycle, the above equipment is set up in an identical manner and the reactor is charged with the filtrate saved from the first cycle above. Fresh bromine (7.63 moles, 393.8 g, 1221 mL) is added and the slurry is stirred and heated to 60° C. The addition funnel is replaced with a heated tube containing molten TMPI (180 g) and equipped with a teflon dip tube to feed the molten TMPI (m.p. = 52 - 53° C, but heated to 60° C), sub-surface, into the Br₂/CH₂Br₂/catalyst slurry, at a temperature of 60° - 70° C, over a period of sixty minutes. The reaction temperature is raised to maintain reflux, as in the first cycle, until a reaction temperature of 94° - 95° C is reached in 1.5 - 2 hours. The reaction mixture is cooled to 50° C, filtered to remove solid product, and the filtrate is recycled to the next (third) batch in an identical manner, adding 7.63 moles of fresh bromine to replace the amount consumed in the second cycle. The solid product is washed with water and then acetone as above and then dried. The yield of the product from this cycle is expected to be 95% and remain constant in the third cycle.

The process of the invention provides a crude ar-brominated phenyl indane product in yields of 70% or more which has a purity of at least 92% by weight, and usually 95% by weight or more, of Br₈ isomer with no more than 8 wt% of underbrominated isomers and usually only 1 - 5% by weight of the B₇ and lower underbrominated isomers. In contrast, the prior art solvent bromination process produces products of only 87% purity in yields of 50% which contain 10% by weight or more of underbrominated products, even after recrystallization.

## Claims

1. A process for ar-brominating 1,3,3-trimethyl-1-phenyl indane to produce octabromo-1,3,3-trimethyl-1-phenyl indane, said process comprising:
(a) adding said 1,3,3-trimethyl-1-phenyl indane to an organic solvent solution of bromine in the presence of an ar-bromination catalyst at a rate such that the addition is completed within about 1.5 hours, with the proportions of solvent to 1,3,3-trimethyl-1-phenyl indane being selected such that the weight ratio of the former to the latter is less than 20 grams of solvent per gram of 1,3,3-trimethyl-1-phenyl indane, and then
(b) heating the reaction mixture to a temperature of at least 90°C so as to produce said octabromo-1,3,3-trimethyl-1-phenyl indane.

2. The process of claim 1 wherein the molar ratio of bromine to 1,3,3-trimethyl-1-phenyl indane fed in the process is in slight excess to the stoichiometric amount of bromine needed to attain the desired degree of bromination.

3. The process of claim 1 wherein the 1,3,3-trimethyl-1-phenyl indane is added as an organic solvent solution.

4. The process of claim 1 wherein said added 1,3,3-trimethyl-1-phenyl indane is molten.

5. The process of claim 4 wherein said 1,3,3-trimethyl-1-phenyl indane is molten and is added beneath the surface of the reaction mixture.

6. The process of claim 1 wherein said weight ratio of solvent to 1,3,3-trimethyl-1-phenyl indane is from 10 to 18 grams/gram.

7. The process of claim 1 including the steps of separating the octabromo-1,3,3-trimethyl-1-phenyl indane from the reaction mixture and then using the remaining reaction mixture along with additional 1,3,3-trimethyl-1-phenyl indane and bromine to repeat steps (a) and (b).

8. The process of claim 7 including adding additional catalyst to said remaining reaction mixture.

9. The process of claim 1 wherein said ar-bromination catalyst is a Lewis acid.

10. The process according to claim 9 wherein said catalyst is selected from the group consisting of iron and halides of iron, aluminum, and zirconium.

## Patentansprüche

1. Verfahren zur Ar-Bromierung von 1,3,3-Trimethyl-1-phenylindan zur Herstellung von Octabrom-1,3,3-trimethyl-1-phenylindan, bei dem:
(a) 1,3,3-Trimethyl-1-phenylindan zu einer organischen Lösungsmittellösung von Brom in Anwesenheit eines Ar-Bromierungskatalysators mit einer solchen Rate gegeben wird, dass die Zugabe innerhalb von etwa 1,5 Stunden vervollständigt ist, wobei die Anteile von Lösungsmittel zu 1,3,3-Trimethyl-1-phenylindan so gewählt sind, dass das Gewichtsverhältnis des ersteren zu dem letzteren weniger als 20 g Lösungsmittel pro Gramm an 1,3,3-Trimethyl-1-phenylindan ist, und dann
(b) die Reaktionsmischung auf eine Temperatur von mindestens 90 °C erhitzt wird, um so das Octabrom-1,3,3-trimethyl-1-phenylindan, herzustellen.

2. Verfahren nach Anspruch 1, bei dem das Molverhältnis von Brom zu 1,3,3-Trimethyl-1-phenylindan, das in das Verfahren eingeführt wird, im leichten Überschuss gegenüber der stöchiometrischen Menge an Brom steht, die benötigt wird, um den gewünschten Bromierungsgrad zu erzielen.

3. Verfahren nach Anspruch 1, bei dem das 1,3,3-Trimethyl-1-phenylindan in Form einer organischen Lösungsmittellösung zugesetzt wird.

4. Verfahren nach Anspruch 1, bei dem das zugesetzte 1,3,3-Trimethyl-1-phenylindan geschmolzen ist.

5. Verfahren nach Anspruch 4, bei dem das 1,3,3-Trimethyl-1-phenylindan geschmolzen ist und unterhalb der Oberfläche der Reaktionsmischung zugeführt wird.

6. Verfahren nach Anspruch 1, bei dem das Gewichtsverhältnis von Lösungsmittel zu 1,3,3-Trimethyl-1-phenylindan 10 bis 18 g/g beträgt.

7. Verfahren nach Anspruch 1, das die Stufe der Trennung des Octabrom-1,3,3-trimethyl-1-phenylindans von der Reaktionsmischung und dann die Verwendung der verbleibenden Reaktionsmischung zusammen mit zusätzlichem 1,3,3-Trimethyl-1-phenylindan und Brom zur wiederholung der Stufen (a) und (b) umfasst.

8. Verfahren nach Anspruch 7, das die Zugabe von zusätzlichem Katalysator zu der verbleibenden Reaktionsmischung umfasst.

9. Verfahren nach Anspruch 1, bei dem der Ar-Bromierungskatalysator eine Lewis-Säure ist.

10. Verfahren nach Anspruch 9, bei dem der Katalysator ausgewählt ist aus der Gruppe bestehend aus Eisen und Halogeniden von Eisen, Aluminium und Zirkonium.

## Revendications

1. Procédé d'aryl-bromation de 1,3,3-tyriméthyl-1-phényl indane pour produire de l'octabromo-1,3,3-triméthyl-1-phényl indane, selon lequel :
(a) on ajoute ledit 1,3,3-triméthyl-1-phényl indane dans une solution de brome dans un solvant organique en présence d'un catalyseur d'aryl-bromation à une vitesse telle que l'addition est achevée en environ 1,5 h, les proportions entre solvant et 1,3,3-triméthyl-1-phényl indane étant choisies de telle façon que le rapport pondéral du premier au dernier soit inférieur à 20 g de solvant par gramme de 1,3,3-triméthyl-1-phényl indane, et ensuite
(b) on chauffe le mélange réactionnel à une température d'au moins 90 °C de façon à produire ledit octabromo-1,3,3-triméthyl-1-phényl indane.

2. Procédé selon la revendication 1, dans lequel le rapport molaire du brome au 1,3,3-triméthyl-1-phényl indane soumis au procédé, est en léger excès par rapport à la quantité stoechiométrique de brome nécessaire pour obtenir le degré de bromation requis.

3. Procédé selon la revendication 1, dans lequel le 1,3,3-triméthyl-1-phényl indane est ajouté sous forme d'une solution dans un solvant organique.

4. Procédé selon la revendication 1, dans lequel ledit 1,3,3-triméthyl-1-phényl indane ajouté, est fondu.

5. Procédé selon la revendication 4, dans lequel ledit 1,3,3-triméthyl-1-phényl indane est fondu, et est ajouté sous la surface du mélange réactionnel.

6. Procédé selon la revendication 1, dans lequel ledit rapport pondéral entre solvant et 1,3,3-triméthyl-1-phényl indane est de 10 à 18 g/g.

7. Procédé selon la revendication 1, comprenant les étapes consistant à séparer l'octabromo-1,3,3-triméthyl-1-phényl indane du mélange réactionnel, et à ensuite utiliser le mélange réactionnel restant avec un supplément de 1,3,3-triméthyl-1-phényl indane et de brome pour répéter les. étapes (a) et (b).

8. Procédé selon la revendication 7, comprenant l'addition d'un supplément de catalyseur dans ledit mélange réactionnel restant.

9. Procédé selon la revendication 1, dans lequel ledit catalyseur d'aryl-bromation est un acide de Lewis.

10. Procédé selon la revendication 9, dans lequel ledit catalyseur est choisi parmi le groupe comprenant le fer et les halogénures de fer, d'aluminium et de zirconium.
